# EUROPÄISCHE PATENTANMELDUNG

(11) **EP 0 879 582 A2**
(43) Veröffentlichungstag der Anmeldung: **25.11.1998**
(21) Anmeldenummer: 98109229.9
(22) Anmeldetag: 20.05.1998
(51) Int. Cl.: A61C 19/00

(54) **Bestrahlungsgerät zur Polymerisation von lichthärtenden Kunststoffen**

(30) Priorität: 21.05.1997 DE 19721311
(71) Anmelder: EKA Gesellschaft für medizinisch-technische Geräte mbH, 82065 Baierbrunn (DE)
(72) Erfinder: Berger, Detlev, 82065 Baierbrunn (DE); Kreitmair, Edith, 81476 München (DE)
(74) Vertreter: Kehl, Günther, Dipl.-Phys.

(57) **Zusammenfassung**

Bestrahlungsgerät zur Polymerisation von lichthärtenden Kunststoffen, insbesondere bei Invivo-Anwendungen im Dentalbereich mit einer Lichtquelle 1 und einer Energieversorgung für die Lichtquelle. Die Lichtquelle umfaßt lichtemittierende Dioden (LED's), die über Batterien oder Akkus betrieben werden.

Gemäß einem weiteren Aspekt der Erfindung sind mehrere LED-Chips 2, die in einem Wellenlängenbereich von 390 bis 520 nm abstrahlen, auf einem gemeinsamen Substrat 6 angeordnet.

## Beschreibung

Die Erfindung betrifft im wesentlichen ein handgeführtes Bestrahlungsgerät zum Aushärten von lichthärtenden Kunststoffen, insbesondere bei Invivo-Anwendungen im Dentalbereich z.B. bei Zahnfüllungen. Lichthärtende Kunststoffe finden eine immer breitere medizinisch-technische Anwendung, wobei die Aushärtung dieser Kunststoffe durch Lichtanregung erfolgt, im Dentalbereich hauptsächlich durch Verwendung von Halogenlampen mit Kaltlichtreflektoren. Diese halogenlampenbetriebenen Bestrahlungsgeräte haben jedoch den Nachteil, daß sie eine große Hitzeentwicklung der Lampen verkraften müssen. Wegen der geringen Effizienz von Halogenlampen im blauen Spektralbereich ist es nicht möglich, handliche batteriebetriebene Bestrahlungsgeräte mit ausreichender Lichtleistung zu erzeugen.

Aus der DE-34 11 996 A1 ist eine Bestrahlungseinheit mit einer Halogen-Reflektorlampe und einem Kühlventilator bekannt geworden.

Aus der DE 42 33 870 A1 ist eine Schutzvorrichtung für ein medizinisches Bestrahlungsgerät bekannt, das ein Fenster aufweist, das für Strahlung im Wellenlängenbereich von 370 bis 520 nm wenigstens teilweise durchlässig ist.

In der prioritätsälteren, jedoch nicht vorveröffentlichten DE 196 19 154 A1 ist ein Bestrahlungsgerät zur Aushärtung von Kunststoffen beschrieben, das mindestens eine lichtemittierende Diode zur Aussendung einer Strahlung im Wellenlängenbereich von etwa 320 nm bis 550 nm aufweist.

Aufgabe der vorliegenden Erfindung ist es, ein effektiv arbeitendes Bestrahlungsgerät im blauen Spektralbereich ohne Hitzeentwicklung und mit so hoher Effizienz zu erzeugen, daß ein Betrieb in einem kleinen handlichen Gehäuse entweder über eine dünne Zuleitung oder sogar mit kleinen Batterien oder Akkus möglich ist.

Überraschenderweise hat sich dabei gezeigt, daß mit blauen LED's eine gute Polymerisation speziell im Dentalbereich bei Invivo-Anwendungen möglich ist. Besonders gut geeignet erscheinen LED's mit geringem Abstrahlungswinkel und mit hoher Beleuchtungsstärke im blauen Wellenlängenbereich z.B. bei 435,450 bzw. 470 nm. Besonders vorteilhaft erwies sich, wenn mehrere LED's zu einem Bündel zusammengefaßt wurden, insbesondere wenn sie so im Winkel gestellt waren, daß ein kleiner Leuchtfleck ausgeleuchtet wurde und damit direkt auf das Objekt gestrahlt wurde. Ebenfalls gute Ergebnisse wurden erzielt, wenn die Strahlung der LED's in ein Lichtleiterbündel eingekoppelt wurde, sei es daß jede einzelne LED in eine Einzelfaser einfokussiert wurde und die Einzelfasern anschließend gebündelt werden bzw. mehrere LED's direkt in ein Lichtleiterbündel einfokussiert wurden.

Am geeignetsten waren Bestrahlungsvorrichtungen, bei denen mehrere LED's mit verschiedenen Wellenlängen verwendet wurden, so daß ein breiterer Spektralbereich abgedeckt wurde. Als vorteilhafteste Anordnung hat sich erwiesen, wenn mehrere lichtemittierende Chips auf einem Substrat möglichst dicht angeordnet sind. Vorteilhafterweise ist eine solche Bündelung mit einer Platte z.B. aus Glas- oder einer Saphirplatte abgedeckt. Eine weitere Verbesserung wurde erreicht, wenn die LED's im Pulsbetrieb gefahren wurden. Ebenso erhöht sich die Lichtabstrahlung, wenn die LED's mit Überlast betrieben werden.

Da der Energiebedarf von LED's sehr gering ist, ist ein effektives Betreiben des Bestrahlungsgerätes mit einer kleinen Batterie bzw. einem kleinen Akku (Monozellen) möglich. Vorteilhafterweise ist in dem Bestrahlungsgerät ein Zeitgeber und eine Stabilisierung nebst einem Handschalter eingebaut. Ebenfalls bewährt hat sich, die LED's zu kühlen, um ein Abfallen des Wirkungsgrades zu vermeiden. Als Kühlung kommen vorzugsweise Minilüfter, Druckluft bzw. Peltierelemente in Betracht.

Die Erfindung wird im folgenden an Hand der in den Figuren schematisch dargestellten Ausführungsbeispiele näher erläutert. Es zeigt:
- Figur 1:: Eine Lichtquelle für ein erfindungsgemäßes Bestrahlungsgerät in der Draufsicht.
- Figur 2:: Einen Schnitt längs der Schnittlinie II-II der Figur 1.
- Figur 3:: Eine weitere Ausführungsform einer Lichtquelle eines erfindungsgemäßen Bestrahlungsgerät in der Draufsicht.
- Figur 4:: Einen Schnitt längs der Schnittlinie IV-IV.
- Figur 5:: Ein Bestrahlungsgerät gemäß einem ersten Ausführungsbeispiel der Erfindung
- Figur 6:: Ein Bestrahlungsgerät gemäß einem zweiten Ausführungsbeispiel der Erfindung

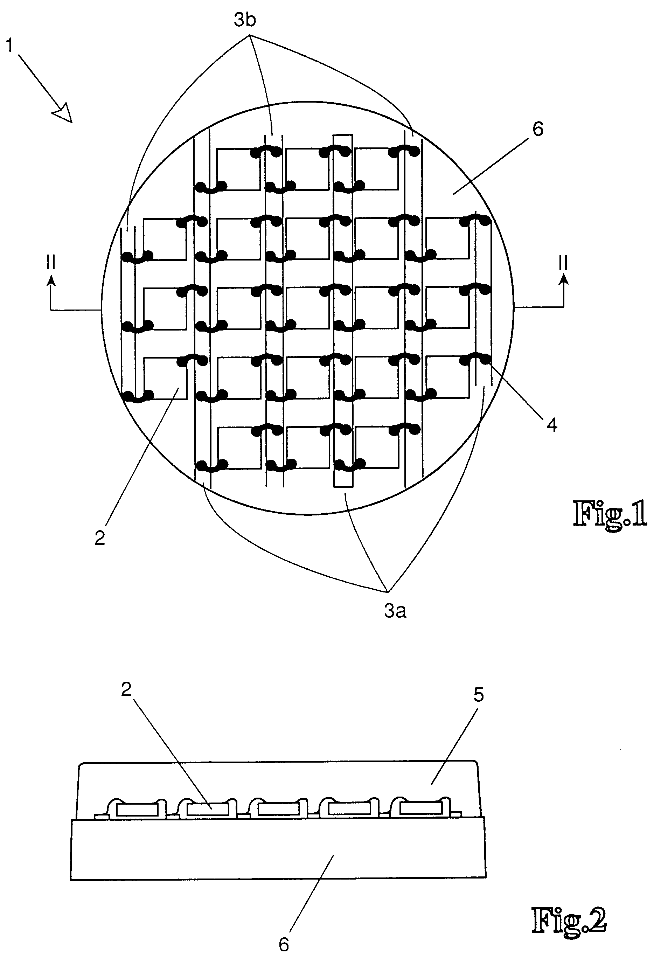

Die in den Figuren 1 bis 4 dargestellte Lichtquelle 1 ist etwa im Maßstab 10:1 vergrößert dargestellt. Die Darstellung ist jedoch nicht notwendigerweise in allen Details maßstabsgetreu.

Wie in Figur 1 zu erkennen ist, sind auf einem aus Al₂O₃ bestehenden Substrat 6 insgesamt fünf Reihen LED-Chips 2 angeordnet. Die LED-Chips 2 bestehen aus Siliziumscheiben mit etwa 1 mm Kantenabmessung. Die LED-Chips sind an Gruppen von Leiterbahnen 3a und 3b über sogenannte "Bonderings" 4 gekoppelt. Durch (nicht dargestellte) Drahtbrücken sind die Leiterbahngruppen 3a jeweils mit dem Pluspol einer Spannungsquelle verbunden, während die Leiterbahngruppen 3b mit dem Minuspol der Spannungsquelle verbunden sind (nicht dargestellt).

Figur 2 zeigt das Substrat 6, das für eine gute Wärmeableitung der von den LED-Chips erzeugten Wärme sorgt. Die LED-Chips 2 sind unter einem (gemeinsamen) Glaskörper 5 angeordnet.

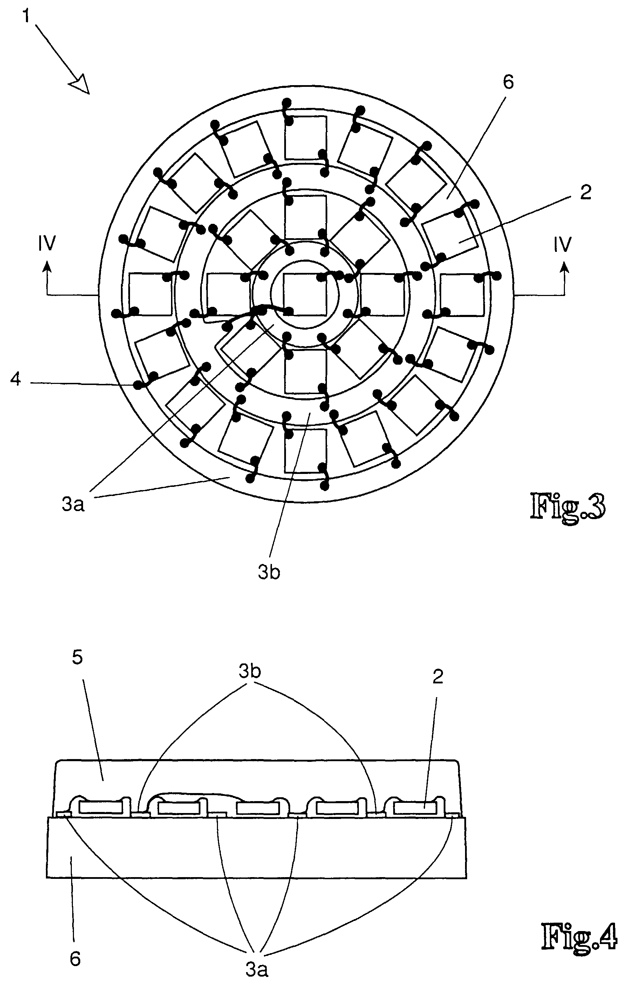

Die Ausführungsform der Figur 3 zeigt eine ähnliche Lichtquelle 1, bei der jedoch die verschiedenen LED-Chips 2 in zwei konzentrischen Kreisen (mit einem Chip in der Mitte) angeordnet sind. Die Leiterbahnen 3a und 3b verlaufen ebenfalls konzentrisch. Figur 4 zeigt die Lichtquelle wiederum im Schnitt längs der Schnittlinie IV-IV.

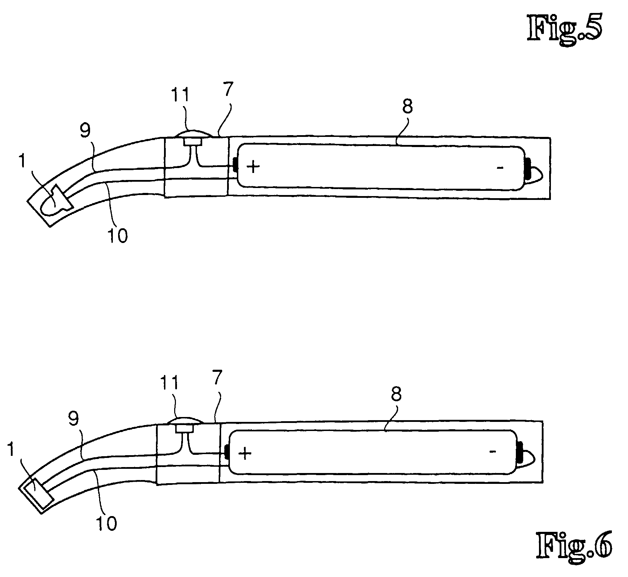

Figur 5 zeigt ein Bestrahlungsgerät gemäß der Erfindung. Es umfaßt ein Gehäuse 7 mit einem Batteriefach 8 und einer in der Spitze des Gehäuses 8 angeordneten Lichtquelle 1. Diese ist mit dem Batteriefach 8 über zwei Drähte 9 und 10 und einen Handschalter 11 verbunden. Bei der Lichtquelle 1 handelt es sich um eine einzelne LED mit einer Emissionswellenlänge im Bereich zwischen 390 und 520 nm.

Figur 6 zeigt eine weitere Ausführungsform, die sich von der in Figur 5 dargestellten Ausführungsform dadurch unterscheidet, daß anstelle einer einzelnen LED eine Lichtquelle 1 vorgesehen ist, die aus mehreren auf einem gemeinsamen Substrat angeordneten LED-Chips, wie in Figur 1 gezeigt, besteht.

Statt jeweils nur einer einzigen Lichtquelle, wie in den Figuren 5 und 6 gezeigt, können auch -sofern der Platz ausreicht - mehrere Lichtquellen vorgesehen sein, die gebündelt werden können.

## Patentansprüche

1. Bestrahlungsgerät zur Polymerisation von lichthärtenden Kunststoffen, insbesondere bei Invivo-Anwendungen (Zahnfüllungen) im Dentalbereich, versehen mit mindestens einer Lichtquelle (1) und einer Energieversorgung für die Lichtquelle, dadurch gekennzeichnet, daß die Lichtquelle (1) aus mindestens einer im blauen Wellenlängenbereich von 390 bis 520 nm abstrahlenden LED besteht und daß die Energieversorgung über Batterien oder Akkus erfolgt.

2. Bestrahlungsgerät zur Polymerisation von lichthärtenden Kunststoffen, insbesondere bei Invivo-Anwendungen (Zahnfüllungen) im Dentalbereich, versehen mit mindestens einer Lichtquelle (1) und einer Energieversorgung für die Lichtquelle, dadurch gekennzeichnet, daß die Lichtquelle (1) mehrere im blauen Wellenlängenbereich von 390 bis 520 nm abstrahlende LED-Chips (2) aufweist, die auf einem gemeinsamen Substrat (6) angeordnet sind.

3. Bestrahlungsgerät nach Anspruch 1 oder 2, dadurch gekennzeichnet, daß mehrere Lichtquellen (1) verwendet werden.

4. Bestrahlungsgerät nach Anspruch 1 oder 3, dadurch gekennzeichnet, daß die Lichtquellen (1) gebündelt werden.

5. Bestrahlungsgerät nach einem der Ansprüche 1 bis 4, dadurch gekennzeichnet, daß mehrere LED's bzw. LED-Chips mit verschiedenen Wellenlängen z.B. 435, 450 und 470 nm verwendet werden.

6. Bestrahlungsgerät nach Anspruch 1, dadurch gekennzeichnet, daß mehrere lichtemittierende Chips (2) (LED-Chips) auf ein Substrat (6) aufgebracht sind.

7. Bestrahlungsgerät nach Anspruch 6, dadurch gekennzeichnet, daß mehrere lichtemittierende Chips (2) mit verschiedenen Wellenlängen auf ein gemeinsames Substrat aufgebracht sind.

8. Bestrahlungsgerät nach einem der Ansprüche 1 bis 7, dadurch gekennzeichnet, daß die LED's gepulst betrieben werden.

9. Bestrahlungsgerät nach einem der Ansprüche 1 bis 8, dadurch gekennzeichnet, daß die LED's mit einer für das emittierte Licht transparenten Scheibe abgedeckt sind.

10. Bestrahlungsgerät nach einem der Ansprüche 1 bis 9, dadurch gekennzeichnet, daß das emittierte Licht in Lichtleiter einfokussiert wird.
